# FASCICULE DE BREVET EUROPEEN

(11) **EP 0 974 312 B1**
(45) Date de publication et mention de la délivrance du brevet: **09.04.2008**
(21) Numéro de dépôt: 99401722.6
(22) Date de dépôt: 08.07.1999
(51) Int. Cl.: A61F 2/02

(54) **Implant de reconstruction**
Rekonstruktionsimplantat
Reconstruction implant

(30) Priorité: 16.07.1998 FR 9809112
(43) Date de publication de la demande: 26.01.2000
(73) Titulaire: Perouse Plastie, 60540 Bornel (FR)
(72) Inventeur: Perouse, Eric, 95290 L'isle Adam (FR)
(74) Mandataire: Blot, Philippe Robert Emile

(56) Documents cités:
- WO-A-95/23565

## Description

La présente invention concerne un implant de reconstruction de forme prédéterminée, du type comportant une enveloppe externe déformable dans laquelle est contenu un liquide de remplissage dont la viscosité est sensiblement égale à celle de l'eau. Un tel implant est connu du document WO-A-9523565.

De tels implants sont utilisés par exemple pour le remplacement chez l'être humain d'une glande dont l'ablation a été rendue nécessaire suite à une maladie ou un accident. De tels implants sont destinés notamment à remplacer un testicule ou une glande mammaire.

D'autres implants de reconstruction du type précité sont utilisés pour le remplacement d'un muscle abîmé, ou encore l'augmentation artificielle du volume apparent de celui-ci.

Les implants testiculaires connus comportent généralement une enveloppe externe déformable réalisée en silicone réticulé. Cette enveloppe définit un logement clos dans lequel est confiné un gel de silicone.

Dans les implants connus, l'épaisseur de l'enveloppe est comprise entre 0,4 et 0,7 mm. En pratique, l'épaisseur de l'enveloppe est aussi réduite que possible, la consistance du testicule étant obtenue par la viscosité du gel contenu dans l'enveloppe. La viscosité d'un tel gel est de l'ordre de quelque Pascal.seconde.

Par suite d'une dégradation de l'image du gel de silicone aux yeux du grand public, les implants, notamment les testicules artificiels, ont été emplis de sérum physiologique en remplacement du gel de silicone.

La viscosité du sérum physiologique est proche de celle de l'eau. Ainsi, afin d'assurer une consistance à la palpation satisfaisante de l'implant, la pression du sérum physiologique dans l'enveloppe a été accrue.

Dans ces conditions, l'enveloppe fine dont l'épaisseur est comprise entre 0,4 et 0,7 mm est maintenue tendue sous l'action de la surpression du sérum physiologique. La turgescence de l'implant ainsi obtenue permet de re produire assez fidèlement la forme et la consistance de l'organe remplacé.

L'existence d'une surpression dans l'enveloppe est à l'origine d'un vieillissement prématuré de l'implant. En effet, il est constaté une diminution progressive de la pression sous l'action d'une migration du sérum physiologique au travers de l'enveloppe. La consistance et la forme de l'implant se dégradent donc avec le temps.

L'invention a pour but de fournir un implant de reconstruction mettant en oeuvre un liquide de remplissage dont la viscosité est sensiblement égale à celle de l'eau, et dont la consistance est durablement proche de celle de l'organe à remplacer.

A cet effet, l'invention a pour objet un implant de reconstruction de forme prédéterminée, du type précité, caractérisé en ce que l'épaisseur de ladite enveloppe est suffisante pour conférer à ladite enveloppe ouverte une forme sensiblement identique à ladite forme prédéterminée en l'absence de liquide de remplissage, et en ce que la pression du liquide de remplissage contenue dans ladite enveloppe fermée est sensiblement égale à la pression hors de l'enveloppe.

Selon le mode particulier de réalisation, l'implant de reconstruction comporte l'une ou plusieurs des caractéristiques suivantes:
- l'épaisseur de l'enveloppe est choisie pour que le rapport de la plus grande dimension de l'implant sur l'épaisseur de l'enveloppe soit comprise entre 2,3 et 20;
- l'épaisseur de l'enveloppe est choisie pour que le rapport de la plus grande dimension de l'implant sur l'épaisseur de l'enveloppe soit comprise entre 5 et 18;
- l'enveloppe comporte une membrane de silicone dont l'épaisseur est supérieure à 2,5 mm;
- l'épaisseur de l'enveloppe est comprise entre 2,5 et 7,5 mm;
- la plus grande dimension de l'implant est comprise entre 2 et 5 cm;
- le liquide de remplissage est du sérum physiologique; et
- ladite forme prédéterminée est une forme ovoïdale et correspond à la forme d'un testicule.

L'invention sera mieux comprise à la lecture de la description qui va suivre, donnée uniquement à titre d'exemple et faite en se référent aux dessins sur lesquels:
- la figure 1 est une vue en perspective d'un testicule selon l'invention;
- la figure 2 est une vue en coupe longitudinale du testicule de la figure 1;
- les figures 3 à 6 sont des vues en coupe longitudinale de variantes de réalisation d'un testicule selon l'invention;
- la figure 7 est une vue en perspective de deux demi-coquilles destinées à être assemblées pour former un testicule selon l'invention; et
- la figure 8 est une vue en perspective du testicule assemblé.

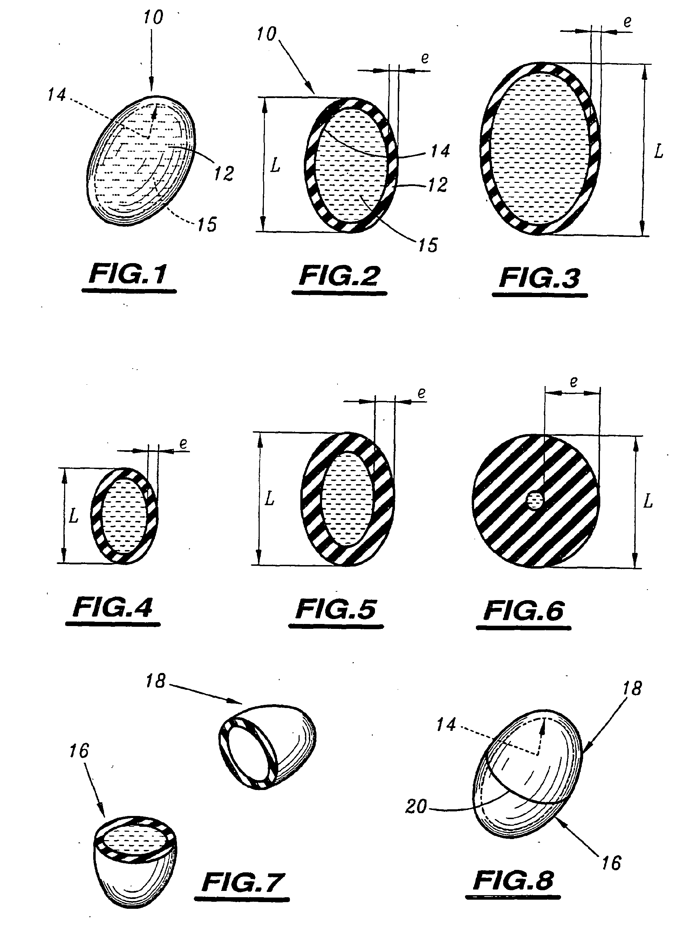

Le testicule 10 représenté sur la figure 1 a une forme ovoïdale. Il comporte une enveloppe épaisse 12 délimitant un logement clos 14 empli d'un liquide de remplissage 15. Le liquide de remplissage 15 est avantageusement du sérum physiologique. Sa viscosité du liquide 15 est sensiblement égale à celle de l'eau, c'est-à-dire de l'ordre de 10⁻³ Pa.s. Il est confiné dans le logement 14 en l'absence d'air à une pression sensiblement égale à la pression atmosphérique.

Le testicule 10 est représenté en coupe sur la figure 2. Suivant sa plus grande dimension, le testicule 10 a une longueur L égale à 3,5 cm . La membrane formant l'enveloppe 12 a une épaisseur constante e égale à 2,5 mm. L'enveloppe est réalisée en un matériau polymère, notamment du silicone réticulé.

L'épaisseur de la membrane formant l'enveloppe 12 est suffisante pour conférer à l'enveloppe ouverte une forme sensiblement identique à la forme finale du testicule en l'absence de liquide de remplissage.

Ainsi, pour une enveloppe ouverte, c'est-à-dire une enveloppe emplie d'air et percée d'au moins un orifice permettant la circulation de l'air, l'enveloppe 12 définit une coque auto-portante reproduisant la forme de l'implant, lorsque aucune contrainte extérieure autre que la pression atmosphérique est appliquée.

A cet effet, l'enveloppe comporte une membrane de silicone dont l'épaisseur e est supérieure à 2,5 mm. Avantageusement, l'épaisseur de l'enveloppe est comprise entre 2,5 et 7,5 mm.

Afin d'assurer une tenue satisfaisante de l'enveloppe, l'épaisseur de celle-ci est choisie pour que le rapport de la plus grande dimension L de l'implant, sur l'épaisseur e de l'enveloppe soit comprise entre 2,3 et 20, et avantageusement entre 5 et 18.

Sur les figures 3 à 6 sont représentés différents testicules de dimensions et d'épaisseurs d'enveloppe variables.

Dans le cas de testicules humaines, leur dimension la plus grande dimension est comprise entre 2 et 5 cm.

Sur la figure 3 est représenté un testicule de plus grande dimension L égale à 4,5 cm. L'épaisseur e de l'enveloppe est de 2,5 mm. Pour un tel testicule, le rapport de la plus grande dimension L sur l'épaisseur e est égal à 18. Malgré ce rapport élevé, l'enveloppe est suffisamment rigide pour imposer la forme du testicule ouvert en l'absence de liquide de remplissage.

Un tel maintien en forme de l'enveloppe est observé pour des rapports de la plus grande dimension de l'implant sur l'épaisseur e de l'enveloppe inférieure à 20.

Sur la figure 4 est représenté un testicule de petite dimension, dont la plus grande dimension L est égale à 2,5 cm. L'épaisseur e de la paroi est de 2,5 mm.

Un tel testicule, empli de sérum physiologique à la pression atmosphérique présente une dureté supérieure au testicule de la figure 3 lors de la palpation. En effet, le rapport de la dimension maximale L sur l'épaisseur e est supérieur.

Sur les figures 5 et 6 sont représentés deux autres exemples de testicules, dont la plus grande dimension L est égale à 3,5 cm. Leur épaisseur e sont respectivement de 5 mm et sensiblement 15 mm. Ils sont tous deux emplis de sérum physiologique à la pression atmosphérique

Le testicule de la figure 6 présente au toucher une dureté supérieure à celui de la figure 5 du fait de la plus grande épaisseur e de l'enveloppe, alors que les deux testicules ont la même dimension maximale.

On conçoit ainsi que la dureté du testicule peut être accrue en augmentant l'épaisseur de l'enveloppe e, sans qu'il soit nécessaire de modifier la pression du liquide de remplissage, celle-ci étant toujours égale à la pression atmosphérique.

L'absence de suspension dans le logement 14 est rendue possible par la rigidité donnée par l'enveloppe épaisse 12 qui forme une coque. Ainsi, la forme et dureté d'un implant selon l'invention sont fournies essentiellement par l'enveloppe 12 et non par le liquide de remplissage. C'est pourquoi, l'épaisseur de la membrane formant l'enveloppe doit avoir une épaisseur suffisante pour garantir la stabilité du profil de l'implant en l'absence de liquide de remplissage alors que le logement 14 est ouvert.

Avec de tels implants, le liquide de remplissage étant à la pression atmosphérique, aucune migration ne se produit au travers de l'enveloppe. Ainsi, la dureté de l'implant à la palpation reste constante au cours du temps.

Pour la fabrication d'un testicule selon l'invention, on réalise initialement deux demi-coquilles 16,18 identiques représentées sur la figure 7. Ces demi-coquilles sont formées par moulage. Elles ont chacune une forme sensiblement hémisphérique.

Comme représenté sur la figure 8, les demi-coquilles 16, 18 sont liées l'une à l'autre pour former l'enveloppe 12. Elles sont assemblées par exemple par collage suivant un plan de joint équatorial 20.

Le logement clos 14 défini entre les deux demi-coquilles 16, 18 est ensuite totalement empli de sérum physiologique à l'aide d'une seringue. Ce remplissage s'effectue depuis la partie inférieure du testicule, un évent étant pratiqué au sommet du testicule afin de permettre l'évacuation de l'air contenu dans le logement.

Le passage de l'aiguille de la seringue d'injection et l'évent sont obturés après remplissage total du testicule à l'aide de gouttes d'élastomère réticulable à froid.

Suivant un autre mode de réalisation, l'enveloppe du testicule est réalisée d'une seule pièce par trempages successifs d'un noyau fusible dans un bain de silicone réticulable à froid. Après extraction du noyau fusible, le testicule est empli de sérum physiologique de manière analogue à celle exposée précédemment.

Suivant encore une autre variante, l'enveloppe 12 peut être réalisée par rotomoulage, l'enveloppe étant formée d'un élastomère réticulable à froid ou à chaud.

L'implant selon l'invention est illustré ici par un testicule artificiel. Toutefois, des implants de reconstruction, en forme de mollet, ou tout autre muscle peuvent être fabriqués avec une structure selon l'invention.

## Revendications

1. Implant de reconstruction (10) de forme prédéterminée, du type comportant une enveloppe externe déformable (12) dans laquelle est contenu un liquide de remplissage (15) dont la viscosité est sensiblement égale à celle de l'eau, **caractérisé en ce que** l'épaisseur de ladite enveloppe (12) est suffisante pour conférer à ladite enveloppe ouverte une forme sensiblement identique à ladite forme prédéterminée en l'absence de liquide de remplissage, et **en ce que** la pression du liquide de remplissage contenue dans ladite enveloppe (12) fermée est sensiblement égale à la pression hors de l'enveloppe.

2. Implant de reconstruction selon l'une quelconque des revendications précédentes, **caractérisé en ce que** l'épaisseur (e) de l'enveloppe (12) est choisie pour que le rapport de la plus grande dimension (L) de l'implant sur l'épaisseur (e) de l'enveloppe (12) soit comprise entre 2,3 et 20.

3. Implant de reconstruction selon la revendication 2, **caractérisé en ce que** l'épaisseur (e) de l'enveloppe (12) est choisie pour que le rapport de la plus grande dimension (L) de l'implant sur l'épaisseur (e) de l'enveloppe (12) soit comprise entre 5 et 18.

4. Implant de reconstruction selon l'une quelconque des revendications précédentes, **caractérisé en ce que** l'enveloppe (12) comporte une membrane de silicone dont l'épaisseur (e) est supérieure à 2,5 mm.

5. Implant de reconstruction selon la revendication 4, **caractérisé en ce que** l'épaisseur (e) de l'enveloppe (12) est comprise entre 2,5 et 7,5 mm.

6. Implant de reconstruction selon la revendication 4 ou 5, **caractérisé en ce que** la plus grande dimension (L) de l'implant est comprise entre 2 et 5 cm.

7. Implant de reconstruction selon l'une quelconque des revendications précédentes, **caractérisé en ce que** le liquide de remplissage (15) est du sérum physiologique.

8. Implant de reconstruction selon l'une quelconque des revendications précédentes, **caractérisé en ce que** ladite forme prédéterminée est une forme ovoïdale et correspond à la forme d'un testicule.

## Claims

1. Reconstruction implant (10) of predetermined shape, comprising a deformable outer envelope (12) including a filler liquid (15) contained in said outer envelope and having a viscosity substantially equal to that of water, **characterised in that** said envelope (12) has a thickness sufficient to impart to said envelope when open a shape which is substantially identical to said predetermined shape in the absence of filler liquid, and **in that** the pressure of the filler liquid contained in said envelope (12) when closed is substantially equal to the pressure outside the envelope.

2. Reconstruction implant according to any one of the preceding claims, **characterised in that** the thickness (e) of the envelope (12) is selected in such way that the ratio of the greatest dimension (L) of the implant to the thickness (e) of the envelope (12) is from 2.3 to 20.

3. Reconstruction implant according to claim 2, **characterised in that** the thickness (e) of the envelope (12) is selected in such a way that the ratio of the greatest dimension (L) of the implant to the thickness (e) of the envelope (12) is from 5 to 18.

4. Reconstruction implant according to any one of the preceding claims, **characterised in that** the envelope (12) comprises a silicone membrane, the thickness (e) of which is greater than 2.5 mm.

5. Reconstruction implant according to claim 4, **characterised in that** the thickness (e) of the envelope (12) is from 2.5 to 7.5 mm.

6. Reconstruction implant according to either claim 4 or claim 5, **characterised in that** the greatest dimension (L) of the implant is from 2 to 5 cm.

7. Reconstruction implant according to any one of the preceding claims, **characterised in that** the filler liquid (15) is saline solution.

8. Reconstruction implant according to any one of the preceding claims, **characterised in that** said predetermined shape is an oval shape and corresponds to the shape of a testicle.

## Patentansprüche

1. Rekonstruktionsimplantat (10) mit vorbestimmter Form, der Art, die eine verformbare äußere Hülle (12) aufweist, in welcher eine Füllflüssigkeit (15) enthalten ist, deren Viskosität im Wesentlichen der des Wassers gleicht, **dadurch gekennzeichnet, dass** die Dicke der Hülle (12) ausreicht, damit der offenen Hülle eine Form verliehen wird, die im Wesentlichen mit der vorbestimmten Form in Abwesenheit von Füllflüssigkeit identisch ist, sowie **dadurch**, dass der Druck der in der geschlossenen Hülle (12) enthaltenen Füllflüssigkeit im Wesentlichen dem Druck außerhalb der Hülle gleicht.

2. Rekonstruktionsimplantat gemäß einem der vorherigen Ansprüche, **dadurch gekennzeichnet, dass** die Dicke (e) der Hülle (12) so ausgewählt ist, dass das Verhältnis der größten Abmessung (L) des Implantats zu der Dicke (e) der Hülle (12) zwischen 2,3 und 20 liegt.

3. Rekonstruktionsimplantat gemäß Anspruch 2, **dadurch gekennzeichnet, dass** die Dicke (e) der Hülle (12) so ausgewählt ist, dass das Verhältnis der größten Abmessung (L) des Implantats zu der Dicke (e) der Hülle (12) zwischen 5 und 18 liegt.

4. Rekonstruktionsimplantat gemäß einem der vorherigen Ansprüche, **dadurch gekennzeichnet, dass** die Hülle (12) eine Silikonmembran aufweist, deren Dicke (e) größer ist als 2,5 mm.

5. Rekonstruktionsimplantat gemäß Anspruch 4, **dadurch gekennzeichnet, dass** die Dicke (e) der Hülle (12) zwischen 2,5 mm und 7,5 mm liegt.

6. Rekonstruktionsimplantat gemäß Anspruch 4 oder 5, **dadurch gekennzeichnet, dass** die größte Abmessung (L) des Implantats zwischen 2 cm und 5 cm liegt.

7. Rekonstruktionsimplantat gemäß einem der vorherigen Ansprüche, **dadurch gekennzeichnet, dass** die Füllflüssigkeit (15) ein physiologisches Serum ist.

8. Rekonstruktionsimplantat gemäß einem der obigen Ansprüche, **dadurch gekennzeichnet, dass** die vorbestimmte Form eine eiförmige Form ist und der Form eines Testikels entspricht.
